# EUROPÄISCHE PATENTANMELDUNG

(11) **EP 1 422 649 A2**
(43) Veröffentlichungstag der Anmeldung: **26.05.2004**
(21) Anmeldenummer: 03026433.7
(22) Anmeldetag: 19.11.2003
(51) Int. Cl.: G06F 19/00

(54) **Verfahren zur Einnahmeüberwachung von Medikamenten**

(30) Priorität: 19.11.2002 DE 10253728
(71) Anmelder: Hafner, Dieter, Dr., D-90482 Nürnberg (DE); Lehrer, Eberhard, 90482 Nürnberg (DE); Schreier, Jochen, 90765 Fürth (DE)
(72) Erfinder: Hafner, Dieter, Dr., D-90482 Nürnberg (DE); Lehrer, Eberhard, 90482 Nürnberg (DE); Schreier, Jochen, 90765 Fürth (DE)
(74) Vertreter: Hafner, Dieter, Dr. Dipl.-Phys.

(57) **Zusammenfassung**

Die Erfindung betrifft ein Verfahren zur Einnahmeüberwachung von Medikamenten, insbesondere zur Erfassung von medikamentenrelevanten Compliance-Daten, wobei bei jeder Einnahme eines Medikamentes vom Patienten oder Probanten manuell eine medikamentenzugehörige, maschinenlesbare, die Art des Medikamentes kennzeichnende Information einer Erfassungseinrichtung zugeführt, von dieser erfasst und an eine elektronische Vorrichtung weitergeleitet wird, sowie eine elektronische Speichervorrichtung vorgesehen ist, durch welche die von der Erfassungseinrichtung kommenden arzneimittelspezifischen Daten gekoppelt mit ihren Erfassungszeiten abrufbar abgespeichert werden.

## Beschreibung

Die Erfindung betrifft ein Verfahren zur Einnahme- oder Anwendungsüberwachung von Medikamenten. Insbesondere ist ein Verfahren angesprochen, bei welchem bei welchem medikamentenrelevante Compliance-Daten ermittelt werden können.

Medikamente im Sinne der Erfindung sind Solche in allen möglichen Darreichungsformen, z. B. oral einzunehmende Medikamente, Apullen, Infusionen, Salben, Zäpfchen, Injektionen, Tropfen, Fläschchen, Sirupe und dgl. mehr. Das Verfahren gemäß der Erfindung eignet sich besonders gut zu Studienzwecken, d. h. für klinische oder anwendungsbezogene Studien, ist aber auch für studienneutrale Nutzer geeignet, beispielsweise Personen im Seniorenalter, die eine Vielzahl von Arzneimitten einnehmen müssen, wobei ein die Person beaufsichtigender Arzt die Möglichkeit haben soll, eine gewisse Einnahmetreue des Patienten zu überwachen.

Aus DE 33 35 301 C2 ist ein Arzneimittelbehälter bekannt, der mit einem nach setzbaren Zeitintervallen aktivierbaren Signalgeber kombiniert werden kann. Bei Entnahme eines Arzneimittels aus dem Arzneimittelbehälter wird über eine an dem Arzneimittelbehälter angeordnete Sensorik in Form von durchtrennbaren Leiterbahnen von dem elektronischen Signalgeber registriert, wann eine Arzneimitteldosis aus dem Arzneimittelbehälter entnommen wurde.

Aus DE 35 18 531 C2 ist es bekannt, an einer Vorrichtung zur Aufbewahrung und zeitlich veranlassten Einnahme von Arzneimitteln einen Codeleser anzubringen, der beim Zusammenfügen der Arzneimittelpackung mit dem elektronischen Signalgeber einen an diesem angeordneten Code betreffend das Arzneimittel in die elektronische Vorrichtung einließt. Aus der eingelesenen Information ist jedoch nicht ersichtlich, wann der die Einrichtung benutzende Patient oder Probant das Arzneimittel aus der Packung entnommen und eingenommen hat. Dazu sind gesonderte Sensorvorrichtungen an dem Arzneimittelbehälter vorgesehen.

Aus DE 40 01 645 C2 ist eine Aufnahmevorrichtung für ein Arzneimittelbehältnis bekannt, die mit einem Betätigungselement versehen ist, durch welches Arzneimitteldosen aus dem Arzneimittelbehältnis ausgedrückt werden können. Über eine elektromechanische Positionsmeßeinrichtung ist es möglich, sowohl den Zeitpunkt als auch den Ort des Arzneimittels in der Arzneimittelverpackung, nämlich einer Blisterpackung elektronisch abzufragen und zu speichern.

Aus DE 101 18 313 A1 ist eine Sensorvorrichtung an einem Arzneimittelbehälter bekannt, die nach Art eines verschiebbaren Schlittens über einem als Blister ausgebildeten Arzneimittelbehälter verschiebbar ist und durch die sowohl der Entnahmezeitpunkt als auch der Ort des Arzneimittels im Blister abgefragt und gespeichert werden können. Auch eine derartige Vorrichtung ist zum Zwecke einer Compliance-Forschung, (Einnahmeüberwachungs-Forschung) einsetzbar.

Alle vorstehend erläuterten Vorrichtungen sind zwar auf unterschiedliche Weise geeignet, die Einnahmetreue eines Patienten in Bezug auf ein Arzneimittel zu überwachen und/oder zu speichern. Die Arzneimittelforschung hat aber großes Interesse daran, das Einnahmeverhalten von Patienten oder Probanten in Bezug auf mehrere miteinander oder nacheinander einzunehmende Arzneimittel oder Arzneimittelkombinationen zu untersuchen, was im Hinblick auf die bekannten Vorrichtungen einen relativ großen apparativen Aufwand erfordert. Für jedes Arzneimittel müßte eine gesonderte Vorrichtung vorgesehen werden.

Der Erfindung liegt die Aufgabe zugrunde, ein Verfahren anzugeben, das geeignet ist, auf einfache Weise das Einnahmeverhalten eines Patienten oder Probanten bezogen auf eine Mehrzahl unterschiedlicher Arzneimittel, auch in unterschiedlichen Darreichungsformen und Packungseinheiten, z. B. orale, Salben, Infusionen, Ampullen, Zäpfchen, Injektionen, Säfte, Tropfen und dgl. zu untersuchen. Diese Aufgabe wird durch die Merkmalskombination des Verfahrensanspruches 1 gelöst. Vorteilhafte Weiterbildungen ergeben sich aus dem Unteransprüchen 2-13.

Erfindungsgemäß ist vorgesehen, daß ein Patient oder Probant jedes mal dann, wenn er eine Arzneimitteldosis aus einer Verpackung entnimmt, manuell eine medikamentenzugehörige maschinenlesbare, die Art des Medikaments kennzeichnende Information einer Erfassungseinrichtung zuführt, die mit einer elektronischen Speichervorrichtung verbunden ist. Die von der Erfassungseinrichtung kommenden arzneimittelspezifischen Daten werden gekoppelt mit ihren Erfassungszeiten abgespeichert und können so für Forschungszwecke auf einfache Weise ausgelesen und/oder mit den Einnahmesolldaten oder Studienprofilen korreliert werden. Auf diese Weise kann durch Ausdruck der gespeicherten Daten problemlos ein Einnahmeprofil eines Patienten oder Probanten hinsichtlich einer Vielzahl von unterschiedlichen Arzneimitteln erstellt und untersucht werden. Selbstverständlich muß bei einem derartigen Verfahren immer davon ausgegangen werden, daß ein Patient oder Probant dann, wenn er die medikamentenrelevante maschinenlesbare Information in die Erfassungseinrichtung einließt, das Arzneimittel auch einnimmt. Dies ist aber bei den bekannten Vorrichtungen ebenso, diese können nur feststellen, ob ein Patient oder Probant ein Arzneimittel aus der Arzneimittelverpackung entnommen hat. Eine tatsächliche Einnahmeüberwachung ist auch mit den Vorrichtungen nach dem oben aufgeführten Stand der Technik nicht möglich.

Die Medikamenteninformation ist vorteilhafter Weise eine Codierung, die manuell einer als Codelesevorrichtung ausgebildeten Eingabevorrichtung zugeführt werden kann. Insbesondere sind optische Codierungen, insbesondere Barcodes und dgl. angesprochen, die durch eine optische Lesevorrichtung, z. B. einer Barcodelesevorrichtung auf einfache Weise erfaßt werden. Die Codierung kann auf dem Arzneimittelblister, auf einem Beipackzettel oder auf einem gesonderten Codeträger angeordnet werden und elektronisch optisch oder magnetisch auslesbar sein. Es ist auch möglich, auf dem Codeträger gesondert oder zusätzlich Patientendaten im maschinenlesbarer Form abzuspeichern. Prinzipiell kann als Codierung die auf Arzneimittelpackungen in einigen Ländern vorgeschriebene oder vorgesehene Pharmazentralnummer verwendet werden. Es ist aber auch möglich, eine Codierung durch den behandelnden Arzt zu erstellen, beispielsweise durch einen Barcodedrucker, ein mit dem aufgedruckten Barcode versehenes Klebeetikett kann dann beispielsweise auf einem Codeträger oder der Arzneimittelpackung aufgebracht werden.

Um eine gewisse "Patientenführung" durch das Verfahren zu ermöglichen, können die von der Erfassungseinrichtung kommen den arzneimittelspezifischen Daten in der elektronischen Vorrichtung mit darin abgespeicherten Anwendungs/Einnahme-Solldaten verglichen werden und eine Einnahmeerinnerungsfunktion ausgelöst werden, falls die Erfassungseinrichtung keine arzneimittelspezifischen Daten hinsichtlich eines abgespeicherten Arzneimittels innerhalb einer vorgebbaren Solleinnahmezeit registriert. Vorteilhafterweise gilt dies natürlich für eine Vielzahl von unterschiedlichen Arzneimitteln. Es ist zusätzlich auch möglich, die Daten Nebenwirkungsangaben oder Kontraindikationen über die Arzneimittel oder Arzneimittelkombinationen umfassen, die auf einer Anzeigevorrichtung der elektronischen Vorrichtung anzeigbar sind, wobei eine Wamfunktion ausgeübt werden kann.

Zusätzlich oder ergänzend ist es möglich, z. B. eine Cursorfunktion auf der Anzeigevorrichtung vorzusehen, über die vom Benutzer die Nebenwirkungsanzeigen markiert werden können und durch Eingabe eines Speicherbefehls die Art der Nebenwirkung zusammen mit dem Zeitpunkt Ihres Auftretens in der Vorrichtung abgespeichert werden kann.

Anstelle der Cursorfunktion ist es auch möglich, mit einem berührungssensitiven Display, z. B. durch einen Stift, Nebenwirkungsanzeigen zu markieren und zu speichern.

Der Patient/Probant ist dadurch in die Lage versetzt, bereits während einer Einnahmephase oder Studie Nebenwirkungen zu erfassen und dem die Daten auswertenden Institut oder Arzt zur Verfügung zu stellen.

Der Informationsgehalt der Codierung kann so breit ausgelegt sein, daß die Einnahmesolldaten bei jedem Erfassungsvorgang der Erfassungseinrichtung in der elektronischen Vorrichtung neu abgespeichert werden. Außerdem ist es möglich, das die Codierung Nebenwirkungsinformationen enthält, die ebenfalls beim Einlesen der Informationen in eine Nebenwirkungsdatenbank eingelesen werden. Stellt die Nebenwirkungsdatenbank fest, daß besondere Nebenwirkungen/Kontraindikation aufgrund der Einnahme von zwei Arzneimitteln auftreten, kann die Vorrichtung eine Wamfunktion erzeugen. Als Beispiel sei genannt, daß bei einem Probanten aufgrund einer unregelmäßigen Einnahme einer Arzneimittelkombination z. B. der Blutdruck steigt oder Kopfschmerzen auftreten. Gibt der Patient dies über die Anzeigevorrichtung ein, kann die erzeugte Warenfunktion darauf hinweisen, die Arzneimittelkombination regelmäßiger zu nehmen, zu einem anderen Tageszeitpunkt zu nehmen oder abzusetzen.

Vorteilhafter Weise ist die elektronische Vorrichtung zur Abspeicherung der medikamentenzugehörigen maschinenlesbaren Information und/oder zur Abspeicherung der Nebenwirkungsdatenbank eine mobile, d. h. handhaltbare, am Körper tragbare Vorrichtung.

Die Erfindung ist anhand eines Ausführungsbeispieles in den Zeichnungsfiguren näher erläutert. Diese zeigen:
- Fig. 1:: eine Vorrichtung, mit der das erfindungsgemäße Verfahren durchgeführt werden kann,
- Fig. 2:: eine beispielhafte Displaydarstellung mit einer Menüfunktion zur Anzeige und Auswahl von Nebenwirkungen.

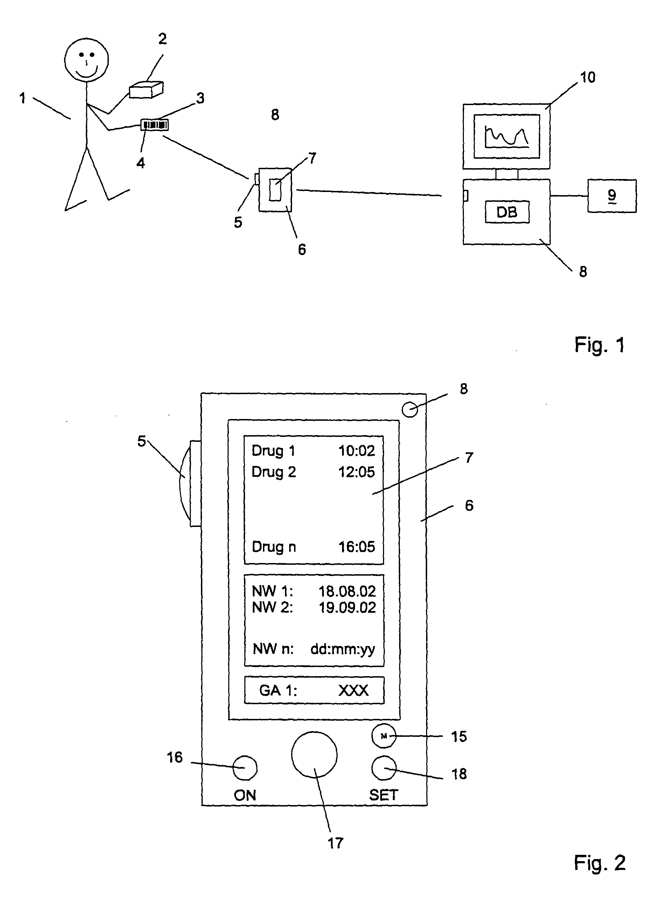

Fig. 1 zeigt in schematischer Darstellung einen Probanten 1, der in der linken Hand eine Arzneimittelpackung 2 und in der rechten Hand einen Codeträger 3 mit einer Strichcodierung 4 hält. Diese Strichcodierung kann ebenso direkt auf der Packung 2 angeordnet sein. Etwa zum Zeitpunkt der Einnahme des aus der Packung 2 zu entnehmenden Arzneimittels, die unterschiedlichste Darreichungsform haben kann, beispielsweise die Form einer Tablette, eines Dragees, eines aufzulösenden Pulvers, Flüssigform aus einem Fläschchen oder einer Ampulle oder als injektierende Flüssigkeit einer Einmalspritze untergebracht ist, führt der Probant 1 den Code 4 einer Erfassungseinrichtung 5 zu, die an einer elektronischen Speichervorrichtung 6 angeordnet oder mit dieser verbunden ist. Die Speichervorrichtung 6 weist eine Anzeige 7 auf, auf der für die Einnahme relevante Daten angezeigt werden können.

Die arzneimittelspezifischen Daten werden gekoppelt mit ihren Erfassungszeiten abgespeichert und an eine elektronische Vorrichtung 8 weitergeleitet, in welcher sie weiterverarbeitet werden können. Die elektronische Vorrichtung 8 kann entweder Bestandteil der Speichervorrichtung sein und ebenso wie die Speichervorrichung zusammen mit dieser als mobile, beispielsweise am Körper tragbare Einheit ausgebildet sein. Es ist aber auch möglich, daß die elektronische Vorrichtung 8 mittels Datenfernübertragung angesprochen wird und in einem Forschungsinstitut eines Pharmauntemehmens steht, so daß dort durch die Speichereinrichtung 6 übertragenen Daten verarbeitet werden können.

Zur Verarbeitung an der elektronischen Vorrichtung, die ein Computer sein kann, sind eine Eingabevorrichtung 9 und ein Display 10 vorgesehen, die Übertragung kann über alle medizinisch möglichen Übertragungswege erfolgen, z. B. IR, Funk, Kabel und dgl.

Die Erfassungseinrichtung 5 ist als Barcodeleser ausgebildet, wenn die Codierung eine optische Codierung in Form eines Barcodes ist. Es ist möglich, die Codierung 4 auf einem Beipackzettel anzuordnen oder auch als elektronisch oder magnetisch auf einem dem Arzneimittel zugehörigen gesonderten Codeträger, sozusagen einer Code-Chip-Karte zu speichern.

Die von der Erfassungseinrichtung 5 kommenden arzneimittelspezifischen Daten werden in der elektronischen Speichervorrichtung 6 mit Solldaten verglichen, wodurch eine Einnahmeerinnerungsfunktion über die Anzeigevorrichtung 7 oder ein akustisches Element 8 ausgelöst werden kann, falls die Speichervorrichtung 6 feststellt, daß innerhalb einer vorgebbaren Solleinnahmezeit oder eines Zeitfensters die auf eine Einnahme hinweisenden arzneimittelspezifischen Daten nicht registriert worden sind.

Die elektronische Speichervorrichtung kann auch eine Mehrzahl von arzneimittelbezogenen Nebenwirkungs- oder Kontraindikationsangaben abgespeichert halten und über das Display 7 anzeigen. Um diese Angaben in die elektronische Speichervorrichtung 6 einzulesen, kann so vorgegangen werden, daß die wesentlichen Angaben Teil der Codierung 4 sind und beim ersten Einlesen sowohl Einnahmesollzeiten hinsichtlich eines ersten Arzneimittels "Drug 1" abgespeichert und angezeigt werden und die entsprechenden Kontraindikations- und Nebenwirkungen ebenfalls in den Speicher eingelesen werden. Sobald alle Codierungen 4, die zu unterschiedlichen Arzneimitteln gehören, ein erstes Mal eingelesen sind, ist die Speichervorrichtung 6 vorprogrammiert. Entweder automatisch oder über eine Menütaste 15 oder eine Starttaste 16 initiierbar läuft nun das Einnahmesollprogramm ab.

Dabei wird angezeigt, daß z. B. das Arzneimittel 1 um 10.02 Uhr das nächste Mal einzunehmen ist, das Arzneimittel 2 um 12.05 Uhr und das Arzneimittel 3 um 16.05 Uhr. Erfolgt keine Einnahme innerhalb eines vorgegebenen Zeitfensters um die Solleinnahmezeit herum, wird über ein akustisches Element 8 ein Alarm ausgelöst. Eine Wamfunktion kann auch über die Anzeige 7 ausgegeben werden.

Ergänzend ist es auch möglich, das die Vorrichtung automatisch bei jedem Einlesevorgang oder auch in regelmäßigen Abständen abfragt, ob zusätzliche Arzneimittel eingenommen worden sind und - wenn ja, diese über das gesonderte Fenster auf dem Display in die Vorrichtung einzulesen.

Über eine Cursortaste 17 können bestimmte Nebenwirkungen, die aufgrund der eingelesenen Codierungen 4 abgespeichert sind hervorgehoben werden, beispielsweise durch einen Rahmen oder durch sonstige optische Mittel und über eine Set-Taste 18 zusammen mit einer Zeitinformation abgespeichert werden. Daneben ist es aber auch möglich, weitere körperliche Empfindungen oder Wahrnehmungen einzugeben und zeitlich abzuspeichern, so daß diese ebenfalls in die im in der Speichervorrichtung 6 zur Verfügung stehenden Daten einfließen und zusammen mit diesen ausgelesen und ausgewertet werden können.

Nebenwirkungen- oder Kontraindikationsangaben können aber auch auf andere Weise in die Speichervorrichtung 6 eingelesen werden, beispielsweise die Übertragung von einem gesonderten Programmierrechner über eine Infrarot-Datenverbindung.

In der Nebenwirkungsdatenbank, die innerhalb der Speichervorrichtung 6 durch erstmaliges Einlesen der Codierung 4 aufgebaut wird, können die Nebenwirkungen unterschiedlicher Arzneimittel miteinander abgeglichen und auf ihre Wechselwirkung hin analysiert werden und bei Auftreten von besonderen Nebenwirkungen die Wamfunktion erzeugt werden.

Die elektronische Speichervorrichtung 6 kann ähnlich wie ein üblicher Personal-Assistant-Computer ausgebildet sein, beispielsweise wie Geräte, die unter dem Handelsnamen "Palm" vertrieben werden. Hinzuzufügen ist lediglich eine Erfassungseinrichtung, die geeignet ist, maschinenlesbare, medikamentenzugehörige Information einzulesen.

### BEZUGSZEICHENLISTE

- 1: Proband
- 2: Arzneimittelpackung
- 3: Codeträger
- 4: Strichcodierung
- 5: Erfassungseinrichtung
- 6: Speichervorrichtung
- 7: Anzeige
- 8: Elektronische Vorrichtung
- 9: Eingabevorrichtung
- 10: Display
- 15: Menütaste
- 16: Starttaste
- 17: Cursortaste
- 18: Set-Taste

## Patentansprüche

1. Verfahren zur Einnahme- oder Anwendungsüberwachung von Medikamenten, insbesondere zur Erfassung von medikamentenrelevanten Compliance-Daten, wobei bei jeder Einnahme- oder Anwendungsüberwachung eines Medikamentes vom Patienten oder Probanten manuell eine medikamentenzugehörige, maschinenlesbare, die Art des Medikamentes kennzeichnende Information einer vom Arzneimittelbehältnis getrennten Erfassungseinrichtung zugeführt, von dieser erfasst und an eine elektronische Vorrichtung weitergeleitet wird, in welcher eine elektronische Speichervorrichtung vorgesehen ist, durch welche die von der Erfassungseinrichtung kommenden arzneimittel- und/oder patientenspezifischen Daten gekoppelt mit ihren Erfassungszeiten abrufbar abgespeichert werden.

2. Verfahren nach Anspruch 1,
**dadurch gekennzeichnet, daß**
die medikamentenzugehörige Information eine Codierung ist, die vom Patienten oder Probanten manuell mit einer als Code-Lesevorrichtung ausgebildeten Eingabevorrichtung in Wirkverbindung gebracht wird.

3. Verfahren nach einem der vorhergehenden Ansprüche,
**dadurch gekennzeichnet, daß**
die Codierung eine optische Codierung, insbesondere ein Barcode ist , die durch eine optische Lesevorrichtung, insbesondere einen Barcode-Leser erfasst wird.

4. Verfahren nach einem der vorhergehenden Ansprüche,
**dadurch gekennzeichnet, daß**
die Codierung auf einem Arzneimittelblister, auf einem Beipackzettel oder einer Arzneimittelpackung angeordnet ist

5. Verfahren nach einem der vorhergehenden Ansprüche,
**dadurch gekennzeichnet, daß**
als Codierung eine optische, elektronische oder magnetische Codierung verwendet wird, die auf einem dem Arzneimittel zugehörigen gesonderten Code-Träger angeordnet ist, und von diesem abgelesen wird.

6. Verfahren nach einem der vorhergehenden Ansprüche,
**dadurch gekennzeichnet, daß**
als Codierung die Pharmazentralnummer verwendet wird.

7. Verfahren nach einem der vorhergehenden Ansprüche,
**dadurch gekennzeichnet, daß**
die Codierung durch den behandelnden Arzt, eine betreuende Apotheke oder ein Studieninstitut erstellt wird.

8. Verfahren nach einem der vorhergehenden Ansprüche,
**dadurch gekennzeichnet, daß**
die von der Erfassungseinrichtung kommenden arzneimittelspezifischen Daten in der elektronischen Speichervorrichtung medikamentenzugehörig mit Anwendungs- oder Einnahmesolldaten verglichen werden die für eine Mehrzahl von unterschiedlichen Arzneimitteln, ggf. in Form eines Studienprofils abgespeichert sind und eine Einnahme-Erinnerungsfunktion ausgelöst wird, falls die Erfassungseinrichtung keine arzneimittelspezifischen Daten hinsichtlich eines abgespeicherten Arzneimittels innerhalb einer vorgebbaren Soll-Einnahmezeit registriert.

9. Verfahren nach einem der vorhergehenden Ansprüche,
**dadurch gekennzeichnet, daß**
über eine Anzeigeeinrichtung der elektronischen Speichervorrichtung arzneimittelbezogenen Nebenwirkungsangaben anzeigbar und auswählbar sind und die Art einer Nebenwirkung vom Benutzer der elektronischen Vorrichtung zusammen mit dem Zeitpunkt Ihres Auftretens abspeicherbar ist.

10. Verfahren nach einem der vorhergehenden Ansprüche,
**dadurch gekennzeichnet, daß**
die medikamentenzugehörige maschinenlesbare Information arzneimittelzugehörige Nebenwirkungsinformationen enthält, die beim Einlesen der Information mittels der Erfassungseinrichtung in eine Nebenwirkungsdatenbank eingelesen werden.

11. Verfahren nach dem vorhergehenden Anspruch,
**dadurch gekennzeichnet, daß**
in der Nebenwirkungsdatenbank die Nebenwirkungen unterschiedlicher Arzneimittel miteinander abgeglichen und auf Ihre Wechselwirkung hin analysiert werden und bei möglichem Auftreten von besonderen Nebenwirkungen aufgrund der Einnahme eine Wamfunktion erzeugt wird.

12. Verfahren nach einem der vorhergehenden Ansprüche,
**gekennzeichnet durch**
die medikamentenzugehörige maschinenlesbare Information arzneimittelzugehörige Gegenanzeigen oder Kontraindikationen enthält, die ein Einlesen der Information mittels der Erfassungseinrichtung in eine Nebenwirkungsdatenbank eingelesen werden.

13. Verfahren nach Anspruch 12,
**dadurch gekennzeichnet, daß**
in der Nebenwirkungsdatenbank die Gegenwirkungen unterschiedlicher Arzneimittel miteinander abgeglichen und auf Ihre Wechselwirkung hin analysiert werden und beim Auftreten von Kontraindikationen aufgrund der Einnahme von zwei Arzneimitteln eine Warenfunktion erzeugt wird.
